# EUROPEAN PATENT SPECIFICATION

(11) **EP 0 639 990 B1**
(45) Date of publication and mention of the grant of the patent: **14.07.2004**
(21) Application number: 94905002.5
(22) Date of filing: 20.01.1994
(51) Int. Cl.: A61L 29/00, C08J 7/04

(54) **A METHOD FOR PRODUCING A HYDROPHILIC COATING ON A SURFACE AND A MEDICAL ARTICLE PRODUCED BY THE METHOD**
VERFAHREN ZUR HERSTELLUNG EINES HYDROPHILEN ÜBERZUGES AUF EINER OBERFLÄCHE UND EIN MITTELS DIESES VERFAHRENS HERGESTELLTER MEDIZINISCHER ARTIKEL
PROCEDE DE PRODUCTION D'UN REVETEMENT HYDROPHILE SUR UNE SURFACE ET ARTICLE MEDICAL PRODUIT SELON CE PROCEDE

(30) Priority: 21.01.1993 DK 7193
(43) Date of publication of application: 01.03.1995
(73) Proprietor: COLOPLAST A/S, 3050 Humlebaek (DK)
(72) Inventor: RODSTEN, Carsten, Bob Brandbjerggaard, DK-4070 Kirke Hyllinge (DK)
(74) Representative: Inspicos A/S
(86) International application number: PCT/DK1994/000035
(87) International publication number: WO 1994/016747

(56) References cited:
- EP-A- 0 093 093
- EP-A- 0 217 771
- EP-A- 0 586 324
- DK-A- 9 200 768

## Description

This invention relates to a method of preparing a hydrophilic coating as described in the introductory part of claim 1.

It is known to coat medical devices, e.g., catheters for introduction into human cavities such as blood vessels, digestive organs and the urinary system, with a hydrophilic coating, as a minimum applied on that part of the surface which gets into contact with the mucous membranes, etc., during introduction of the device. Whereas such coating in dry condition is not particularly smooth, so that the handling of the device does not become inconvenient, when it is moistened with water immediately before introduction into the human body it becomes extremely slippery, thereby providing a substantially painless introduction.

A large number of methods are known for the production of hydrophilic surface coatings.

These methods are mainly based on the fact that the substrate to be provided with a hydrophilic surface coating, in the course of one or more process stages with intermediary drying and curing, is coated with one or more (mostly two) layers, which are caused to react with one another in different ways, e.g., by polymerization initiated by irradiation, by graft polymerization, by the formation of interpolymeric network structures, or by direct chemical reactions. Regarding this subject, please refer to DK-A-900 855, DK-B-159 018, EP-A-379 156, EP-A-454 293, EP-B2-93 093, GB-A-1 600 963, US-A-4 119 094, US-A-4 373 009, US-A-4 729 914, US-A-5 041 100, and US-A-5 120 816, and to WO-A-9005162 and WO-A-9119756.

According to a method known from' US-A-5 001 009, a hydrophilic surface coating is prepared on a substrate by applying, in two stages or in one combined stage, on the substrate a reactive or an adhesive primer layer and then the actual hydrophilic surface layer, which in this case comprises polyvinylpyrrolidone [PVP] as the active constituent. By this method, there is no chemical reaction between the components of the two layers applied.

Where a device of said type, e.g., a catheter, is to remain inside the body only for a short period, there may be a risk that water will be extracted from the hydrophilic surface coating and into the body fluids in the surrounding mucous membranes etc., owing to the higher osmotic potential of said body fluids. As a result of the extraction of water, the hydrophilic surface coating will have a tendency to stick to the surrounding tissues, and the removal of the medical device from the body may be painful.

EP-B-217 771 describes a method of said type for the production of an improved hydrophilic coating that maintains the smoothness of the coating for a longer period of time.

According to this method, on a hydrophilic coating prepared by an already known technique and cured in a separate process stage, an additional and separate coating is applied, consisting of a solution including an osmolality promoting agent and selected from among mono- and disaccharides, sugar alcohols, and nontoxic organic and inorganic salts, whereupon the solvent is allowed to evaporate. As a viscosity controlling agent the solution may also contain a polymer. When dried outside the human body a surface produced according to this method remains moist in a longer period of time than conventionally prepared hydrophilic surface coatings, and catheters with a hydrophilic coating improved in this way will be easier to remove than those with a conventional coating.

As distinct from said method, the method according to the present invention is characterized by the measures stated in the characterizing part of claim 1.

The method according to this invention leads to a simpler production and an improved stability as compared to the known method according to EP-B-217771, since the osmolality promoting agent, without any use of an additional, separate coating step, is included in that solution from which the hydrophilic surface coating or its outermost layer is produced. The osmolality promoting agent is most frequently dissolved in the same solvent as the components for producing the hydrophilic coating, but may also merely be emulgated or suspended therein.

In the method of the invention the osmolality promoting agent may be any compound that ensures the desired equalization of the difference in osmotic pressures between the moistened coating and the surrounding body fluid. In the method according to the invention, the osmolality promoting agent is selected from urea and mixtures hereof, said agent being preferably incorporated into the solution by dissolution or emulsification.

The term "urea" used herein should be understood to comprise urea that has been N-substituted or N,N-disubstituted by lower alkyl.

The preferred embodiment of the method according to the invention in principle relies on process stages corresponding to those described in said US-A-5 001 009, where a primer is first applied to the substrate, for instance a primer containing nitrocellulose applied in a solution. After drying of the primer layer, an outer layer is applied consisting of a solution of polyvinylpyrrolidone in a solvent selected from among tetrahydrofuran, methyl(ene) chloride, toluene, acetone, a lower aliphatic alcohol, cyclohexanone, C₂-C₄-alkyl acetates, butyrolactone, and dimethylformamide, the most important constituent being ethyl alcohol. According to the invention, this solution contains urea as the osmolality promoting agent in a quantity of 1-20 per cent by weight, preferably 2-15 per cent by weight and particularly 3-8 per cent by weight on the basis of dry polyvinylpyrrolidone.

If said content of urea exceeds a value of about 10 per cent by weight, on the basis of dry polyvinylpyrrolidone, a smarting sensation may be felt at the introduction of the catheter, depending on the nature of the hydrophilic coating.

As explained in the following, testing achieved good results with a urea quantity of 5-6 per cent by weight. It was found that a significantly lower amount of urea does not give the desired effect with regard to retention of the water used to moisten the coating before introduction of the device, whereas a significantly larger amount may cause inconveniences in the form of a smarting sensation during the introduction.

The invention also relates to a medical device for introduction into a body cavity, as described in the introductory part of claim 10.

Such devices may especially include catheters, wound drains, and certain surgical instruments. Regardless of the fact that the primary object of this invention is the production of improved catheters for introduction into the urethra in connection with the treatment of dysuria and the achievement of bladder control, the invention embraces all such devices intended for the introduction into and withdrawal from a body cavity, whether for human or veterinary use.

A medical device of said type is, according to the invention, characterized in the features stated in the characterizing part of claim 10.

The example below illustrates the preparation according to the invention of a hydrophilic coating on a catheter based on a polyvinyl chloride [PVC] substrate as well as a test of the coated catheters prepared this way with different contents of urea as the osmolality promoting agent, illustrating the improved retention of water on such produced hydrophilic surfaces.

### EXAMPLE

A catheter made of PVC was prepared according to a modification of the method described in Example 1 in US-A-5 001 009 and coated on part of its surface with a hydrophilic coating with improved water retention by first applying a primer layer by immersion into a mixture of 5.4 g of low-viscosity nitrocellulose, 2 g of dibutylphthalate, and 1.9 g of polyvinyl butyral [PVB], in a mixed solvent comprising isopropanol, ethyl acetate, ethanol, and acetone (36:13:6:25:18:1.5 vol/vol). After drying for 5 minutes at 65°C, the catheter coated as described above was provided with an outer layer by immersion into a solution of 6.6 g of polyvinylpyrrolidone and 5 per cent by weight of urea in relation to polyvinylpyrrolidone in a solvent mixture of ethanol, ethyl acetate, and dimethylformamide (64:23.5:12.5). Finally, the catheter was dried at 65°C for 60 minutes.

Three healthy volunteers (A, B, and C) assessed the resistance when a catheter prepared according to said method was removed after a conventional catheterization, according to the following score system:
1 No resistance
2 Slight resistance
3 Great resistance

The table below shows the results:

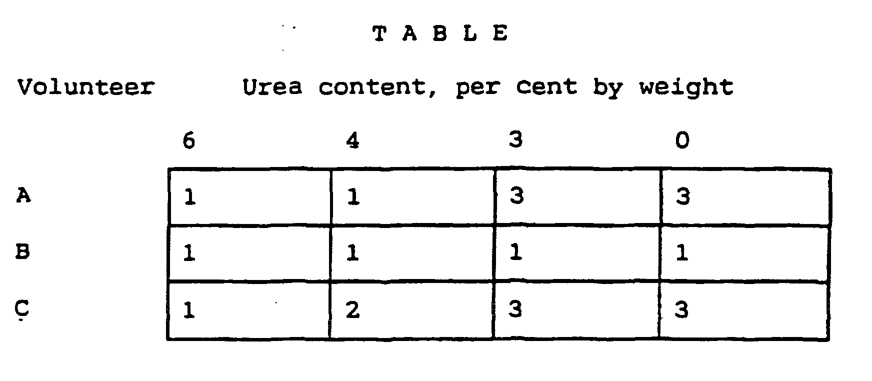

## Claims

1. A method for the preparation of a hydrophilic coating with improved retention of water on a surface, wherein the surface, in one or more process steps, is coated with at least one solution of components, which components will combine to form such a hydrophilic coating, and wherein in the final process step an osmolality promoting agent is applied to the surface, **characterised in that** the osmolality promoting agent is selected from urea which is incorporated into said solution or into the last solution applied of several such solutions and applied in the same process step as this solution.

2. A method according to claim 1, wherein the surface in one process step is coated with one solution of components, which components combine to form said hydrophilic coating, and wherein an osmolality promoting agent is applied to the surface, **characterised in that** the osmolality promoting agent is selected from urea, and is dissolved in or suspended or emulsified into said solution and is applied in the same process step as this solution.

3. A method according to claim 1 or 2, **characterised in that** the osmolality promoting agent is dissolved in said solution.

4. A method according to any of the preceding claims, **characterised in that** the osmolality promoting agent is emulsified into said solution.

5. A method according to any of the preceding claims, wherein said solution comprises polyvinylpyrrolidone or a derivative thereof as the active component.

6. A method according to claim 1 or 2, wherein said solution comprises polyvinylpyrrolidone or a derivative thereof as the active component, **characterised in that** the osmolality promoting agent in the solution comprises urea in a quantity of 1-20% by weight based on the quantity of dry polyvinylpyrrolidone

7. A method according to claim 6, wherein the quantity of urea is 2-15% by weight based on the quantity of dry polyvinylpyrrolidone.

8. A method according to claim 7, wherein the quantity of urea is 3-8% by weight based on the quantity of dry polyvinylpyrrolidone.

9. A method according to any of the preceding claims, wherein a primer is applied to the substrate before the surface is coated with the solution.

10. A medical device for introduction into a body cavity, which device on a least part of its surface has a hydrophilic coating, of which the water retention has been improved by means of an osmolality promoting agent, **characterised in that** said osmolality promoting agent is selected from urea which is incorporated into the hydrophilic coating itself by incorporating the osmolality promoting agent in a solution or in the last solution applied from which the coating is formed.

11. A medical device according to claim 10, which device on a least part of its surface has one hydrophilic coating, **characterised in that** the osmolality promoting agent is incorporated into the hydrophilic coating itsetf by incorporating the osmolality promoting agent in the solution from which the coating is formed.

12. A device according to claim 10 or 11, wherein said solution comprises polyvinylpyrrolidone or a derivative thereof as the active component.

13. A device according to claim 10 or 11, wherein the layer of the coating includes polyvinylpyrrolidone or a derivative thereof, **characterised in that** the osmolality promoting agent comprises urea in a quantity of 1-20% by weight based on the quantity of dry polyvinylpyrrolidone in the hydrophilic layer.

14. A device according to claim 13, wherein the quantity of urea is 2-15% by weight based on the quantity of dry polyvinylpyrrolidone.

15. A device according to claim 14, wherein the quantity of urea is 3-8% by weight based on the quantity of dry polyvinylpyrrolidone.

16. A device according to any one of the claims 10-15, **characterised in that** the medical device is a catheter, mainly intended for introduction into the urethra.

17. A device according to any of the claims 10-16 which is prepared as defined in any of the claims 1-9.

## Patentansprüche

1. Verfahren zur Herstellung einer hydrophilen Beschichtung mit verbesserter Wasserretention auf einer Oberfläche, wobei die Oberfläche, in einem oder mehreren Verfahrensschritten, mit mindestens einer Lösung von Komponenten beschichtet wird, die sich unter Bildung einer solchen Beschichtung vereinigen, und wobei im abschließenden Verfahrensschritt ein die Osmolalität förderndes Mittel auf die Oberfläche aufgebracht wird,
**dadurch gekennzeichnet, dass**
das die Osmolalität fördernde Mittel unter Harnstoff ausgewählt wird, der in diese Lösung oder in die letzte aufgebrachte Lösung von mehreren solchen Lösungen, die im gleichen Verfahrensschritt wie diese Lösung aufgebracht werden, eingebracht wird.

2. Verfahren nach Anspruch 1, wobei die Oberfläche in einem Verfahrensschritt mit einer Lösung von Komponenten beschichtet wird, die sich unter Bildung der hydrophilen Beschichtung vereinigen, und wobei ein die Osmolalität förderndes Mittel auf die Oberfläche aufgebracht wird, **dadurch gekennzeichnet, dass** das die Osmolalität fördernde Mittel unter Harnstoff ausgewählt wird und in dieser Lösung gelöst oder suspendiert oder emulgiert und im gleichen Verfahrensschritt wie diese Lösung aufgebracht wird.

3. Verfahren nach Anspruch 1 oder 2, **dadurch gekennzeichnet, dass** das die Osmolalität fördernde Mittel in der Lösung gelöst wird.

4. Verfahren nach einem der mehreren der vorhergehenden Ansprüche, **dadurch gekennzeichnet, dass** das die Osmolalität fördernde Mittel in der Lösung emulgiert wird.

5. Verfahren nach einem oder mehren der vorhergehenden Ansprüche, wobei die Lösung Polyvinylpyrrolidon oder ein Derivat davon als wirksamen Bestandteil enthält.

6. Verfahren nach Anspruch 1 oder 2, wobei die Lösung Polyvinylpyrrolidon oder ein Derivat davon als wirksamen Bestandteil enthält, **dadurch gekennzeichnet, dass** das die Osmolalität fördernde Mittel in der Lösung Harnstoff in einer Menge von 1 bis 20 Gew.-%, bezogen auf die Menge an trockenem Polyvinylpyrrolidon, umfasst.

7. Verfahren nach Anspruch 6, wobei die Menge des Harnstoffs 2 bis 15 Gew.-% beträgt, bezogen auf die Menge an trockenem Polyvinylpyrrolidon.

8. Verfahren nach Anspruch 7, wobei die Menge des Harnstoffs 3 bis 8 Gew.-% beträgt, bezogen auf die Mengen an trockenem Polyvinylpyrrolidon.

9. Verfahren nach einem oder mehren der vorhergehenden Ansprüche, wobei ein Primer auf das Substrat aufgebracht wird, bevor die Oberfläche mit der Lösung beschichtet wird.

10. Medizinische Vorrichtung zur Einführung in eine Körperhöhle, die mindestens auf einem Teil ihrer Oberfläche eine hydrophile Beschichtung aufweist, deren Wasserretention durch ein die Osmolalität förderndes Mittel verbessert ist, **dadurch gekennzeichnet, dass** das die Osmolalität fördernde Mittel unter Harnstoff ausgewählt ist, der durch Einbringen des die Osmolalität fördernden Mittels in eine Lösung oder in die zuletzt aufgebrachte Lösung, aus der die Beschichtung erzeugt ist, in die hydrophile Beschichtung selbst eingebracht ist.

11. Medizinische Vorrichtung nach Anspruch 10, die auf mindestens einem Teil ihrer Oberfläche eine hydrophile Beschichtung aufweist, **dadurch gekennzeichnet, dass** das die Osmolalität fördernde Mittel durch Einbringen des die Osmolalität fördernden Mittels in die Lösung, aus der die Beschichtung erzeugt ist, in die hydrophile Beschichtung selbst eingebracht ist.

12. Vorrichtung nach Anspruch 10 oder 11, wobei die Lösung Polyvinylpyrrolidon oder ein Derivat davon als wirksamen Bestandteil enthält.

13. Vorrichtung nach Anspruch 10 oder 11, wobei die Schicht der Beschichtung Polyvinylpyrrolidon oder ein Derivat davon enthält, **dadurch gekennzeichnet, dass** das die Osmolalität fördernde Mittel Harnstoff in einer Menge von 1 bis 20 Gew.-%, bezogen auf die Menge an trockenem Polyvinylpyrrolidon in der hydrophilen Schicht, umfasst.

14. Vorrichtung nach Anspruch 13, wobei die Menge des Harnstoffs 2 bis 15 Gew.-%, bezogen auf die Menge an trockenem Polyvinylpyrrolidon, beträgt.

15. Vorrichtung nach Anspruch 14, wobei die Menge des Harnstoffs 3 bis 8 Gew.-%, bezogen auf die Menge an trockenem Polyvinylpyrrolidon, beträgt.

16. Vorrichtung nach einem oder mehreren der Ansprüche 10 bis 15, **dadurch gekennzeichnet, dass** die medizinische Vorrichtung ein Katheter ist, der hauptsächlich zur Einführung in die Harnröhre vorgesehen ist.

17. Vorrichtung nach einem der mehreren der Ansprüche 10 bis 16, die nach einem der Ansprüche 1 bis 9 hergestellt ist.

## Revendications

1. Un procédé de fabrication d'un revêtement hydrophile sur une surface assurant une meilleure rétention de l'eau, dans lequel la surface, en une ou plusieurs étapes, est recouverte d'au moins une solution de composants, lesquels composants s'associent pour former un tel revêtement hydrophile, et dans lequel, lors de l'étape finale du processus, un agent favorisant l'osmolalité est appliqué sur la surface, **caractérisé en ce que** l'agent favorisant l'osmolalité est sélectionné à partir de l'urée qui est incorporée dans ladite solution ou dans la dernière solution appliquée parmi plusieurs de ces solutions et est appliqué lors de la même étape du processus que cette solution.

2. Un procédé selon la revendication 1, dans lequel la surface, lors d'une étape du processus, est recouverte d'une solution de composants, lesquels composants s'associent pour former ledit revêtement hydrophile, et dans lequel un agent favorisant l'osmolalité est appliqué sur la surface, **caractérisé en ce que** l'agent favorisant l'osmolalité est sélectionné à partir de l'urée et est dissous, suspendu ou émulsionné dans ladite solution et est appliqué lors de la même étape du processus que cette solution.

3. Un procédé selon la revendication 1 ou 2, **caractérisé en ce que** l'agent favorisant l'osmolalité est dissous dans ladite solution.

4. Un procédé selon l'une des revendications précédentes, **caractérisé en ce que** l'agent favorisant l'osmolalité est émulsionné dans ladite solution.

5. Un procédé selon l'une des revendications précédentes, dans lequel ladite solution comprend de la polyvinylpyrrolidone ou un de ses dérivés jouant le rôle de composant actif.

6. Un procédé selon la revendication 1 ou 2, dans lequel ladite solution comprend de la polyvinylpyrrolidone ou un de ses dérivés jouant le rôle de composant actif, **caractérisée en ce que** l'agent favorisant l'osmolalité dans la solution comprend de l'urée dont la quantité se situe entre 1 et 20% en poids sur la base de la quantité de polyvinylpyrrolidone sèche.

7. Un procédé selon la revendication 6, dans lequel la quantité d'urée se situe entre 2 et 15% en poids sur la base de la quantité de polyvinylpyrrolidone sèche.

8. Un procédé selon la revendication 7, dans lequel la quantité d'urée se situe entre 3 et 8% en poids sur la base de la quantité de polyvinylpyrrolidone sèche.

9. Un procédé selon l'une des revendications dans lequel une couche primaire est appliquée sur le substrat avant le revêtement de la surface avec la solution.

10. Un article médical devant être introduit dans une cavité du corps, lequel article possède, sur au moins une partie de sa surface, un revêtement hydrophile, dont la rétention de l'eau a été améliorée au moyen d'un agent favorisant l'osmolalité **caractérisé en ce que** l'agent favorisant l'osmolalité est sélectionné à partir de l'urée qui est incorporée dans le revêtement hydrophile lui-même en incorporant l'agent favorisant l'osmolalité dans une solution ou dans la dernière solution appliquée à partir de laquelle le revêtement est formé.

11. Un article médical selon la revendication 10, lequel article possède, sur au moins une partie de sa surface, un revêtement hydrophile, **caractérisé en ce que** l'agent favorisant l'osmolalité est incorporé dans le revêtement hydrophile lui-même en incorporant l'agent favorisant l'osmolalité dans la solution à partir de laquelle le revêtement est formé.

12. Un article selon la revendication 10 ou 11, dans lequel ladite solution comprend de la polyvinylpyrrolidone ou un de ses dérivés jouant le rôle de composant actif.

13. Un article selon la revendication 10 ou 11, dans lequel la couche de revêtement comprend de la polyvinylpyrrolidone ou un de ses dérivés **caractérisée en ce que** l'agent favorisant l'osmolalité comprend de l'urée dont la quantité se situe entre 1 et 20% en poids sur la base de la quantité de polyvinylpyrrolidone sèche dans la couche hydrophile.

14. Un article selon la revendication 13, dans lequel la quantité d'urée se situe entre 2 et 15% en poids sur la base de la quantité de polyvinylpyrrolidone sèche.

15. Un article selon la revendication 14, dans lequel la quantité d'urée se situe entre 3 et 8% en poids sur la base de la quantité de polyvinylpyrrolidone sèche.

16. Un article selon l'une quelconque des revendications de 10 à 15 **caractérisé en ce que** l'article médical est un cathéter, principalement destiné à être introduit dans l'urètre.

17. Un article selon l'une quelconque des revendications de 10 à 16 qui est préparé selon manière définie dans l'une des revendications de 1 à 9.
